# EUROPEAN PATENT SPECIFICATION

(11) **EP 0 489 392 B1**
(45) Date of publication and mention of the grant of the patent: **22.01.1997**
(21) Application number: 91120711.6
(22) Date of filing: 03.12.1991
(51) Int. Cl.: C12Q 1/04, C12N 1/38

(54) **Culture medium supplement for fastidious organisms**
Ein Mediumsupplement für anspruchsvolle Organismen
Supplément d'un milieu de culture pour les organismes exigeantes

(30) Priority: 06.12.1990 US 622874
(43) Date of publication of application: 10.06.1992
(73) Proprietor: Becton Dickinson and Company, Franklin Lakes, New Jersey 07417-1880 (US)
(72) Inventor: Goldenbaum, Paul E., Baltimore, MD 21208 (US); Graziosi, Ann M., Baltimore, MD 21208 (US)
(74) Representative: von Kreisler, Alek, Dipl.-Chem.

(56) References cited:
- EP-A- 0 019 054
- ZENTRALBLATT FüR BAKTERIOLOGIE, MIKROBIOLOGIE UND HYGIENE, vol. 254, no. 1, March 1983, Stuttgart (DE); ABDOU et al., pp. 109-117/
- JOURNAL OF CLINICAL MICROBIOLOGY, vol. 26, no. 4, April 1988, Washington, DC (US); STRATTON et al., pp. 747-749/
- JOURNAL OF CLINICAL MICROBIOLOGY, vol. 25, no. 8, August 1987, Washington, DC (US); WEINSTEIN et al., pp. 1373-1375/
- JOURNAL OF CLINICAL MICROBIOLOGY, vol. 18, no. 5, November 1983, Washington, DC (US); EDBERG et al., pp. 1047-1050/
- WPIL, Week 8801, Derwent Publications Ltd., London (GB); AN 88-005140/

## Description

### 1. Field of the Invention

This invention relates to a fastidious organism supplement, and more particularly to a neutralizing supplement, and to a method for making the supplement.

### 2. Description of Related Art

A polyanionic anticoagulant is an essential ingredient in all blood culture media. Sodium polyanetholesulfonate (SPS) and sodium amylosulfate (SAS) are examples of polyanionic anticoagulants that may be added to blood culture media. SPS, in particular, is routinely added to commercial blood culture media.

SPS has many beneficial effects when used in culture media, such as the inhibition of enzymes, proteins and processes that inactivate the growth of microorganisms. Such enzymes, proteins and processes include phagocytosis, complement, lysozymes and aminoglycoside antibodies. However, SPS is toxic to many strains of organisms, including Neisseria meningitidis, Neisseria gonorrhoeae, Peptostreptococcus anaerobius, Streptobacillus moniliformis, Gardnerella vaginalis, and Mycoplasma sp. A discussion about SPS and the variables influencing the speed and recovery rate of microorganisms is incorporated by reference, C.L. Strand and J.A. Shulman, Bloodstream Infections, 21-44 (1988).

Numerous attempts have been made to neutralize the toxic effect of polyanionic anticoagulants in different media, especially in blood culture media, and to enhance the isolation, growth, recovery and/or detection of fastidious organisms.

The neutralization of SPS by hemoglobin has been reported by S.C. Edberg, et al. in "Inactivation of the Polyanionic Detergent Sodium Polyanetholesulfonate by Hemoglobin." J. Clin. Microbiol. 18:1047-1050 (1983). It was reported that hemoglobin can inactivate SPS but since hemoglobin is one of the major constituents inside blood culture bottles, and since by lysis its concentration can change over time, it is extremely difficult to predict whether the amount of free hemoglobin will be enough to inhibit a particular isolate of Neisseria in an individual blood culture bottle.

The neutralization of SPS by gelatin (typically 1.0 to 1.2% w/v) has been reported by M. Weinstein, et. al. in "Controlled Evaluation of Modified Radiometric Blood Culture Medium Supplemented with Gelatin for Detection of Bacteriemia and Fungemia." J. Clin. Microbiol. 25:1373-1375 (1987). The presence of gelatin in SPS-containing blood culture medium was shown to significantly inhibit the recovery of a variety of fastidious organisms, including staphylococci and members of the Enterbacteriaceae family.

U.S. Patent No. 4,217,411 to Le Frock, et al. discloses enrichment for enhancing the detection and growth of the etiological agents of bacteriemia comprising Fildes peptic digest of blood and certain other compounds. The enrichment comprises Fildes peptic digest, Vitamin B₁₂, NAD, L-Glutamine, Co-enzyme A, Pyruvate, Catalase, Glutamate, Vitamin K₁, Co-Carboxylase and Cysteine hydrochloride. The enrichment supports growth of a number of organisms, but does not contain a neutralizing effect for polyanionic anticoagulants.

The addition of gelatin to blood culture media to prevent inhibition of various organisms caused by SPS has been suggested by Reimer, et al., in "Controlled Evaluation of Trypticase Soy Broth with and without Gelatin and Yeast Extract in the Detection of Bacteriemia and Fungemia." Diagn. Microbiol. Infect. Dis. 8:19-24 (1987). However, the addition of gelatin without sufficient growth factors was found not to enhance the growth of fastidious organisms.

It was concluded that blood culture media could not be used for non-blood samples by Abdou, et al., in "Unsuitability of Blood Culture Media Containing Sodium Polyanetholesulfonate for the Detection of Fastidious Microorganisms in CSF and Other Blood-Free Body Fluids." Zbl. Bakt. Hyg., I. Abt. Orig. A 254, 109-117 (1983). It was found that SPS containing media could not be used for culturing cerebral spinal fluid and other blood-free body fluids and that media without anticoagulants could not be used to culture blood.

Fastidious organisms in clinical microbiology, are difficult to isolate or cultivate on ordinary media because of their need for special nutritional factors. In particular, factor V and factor X are nutritional factors which stimulate the growth of a number of fastidious bacteria including Haemophilus influenzae, Haemophilus parainfluenzae and Gardnerella vaginalis. Since factors X and V cannot be made by fastidious organisms in a culture media, they need to be supplied nutritionally to a culture media for proper growth of the fastidious organisms.

Factor X is haemin (hemin), a complex organic molecule which is present in hemoglobin in red blood cells. Several species of Haemophilus are examples of bacteria which require factor X or haemin for growth.

Factor V is nicotinamide adenine dinucleotide, or NAD, a large complex organic molecule which is required for the growth of several fastidious bacteria, including some species of Haemophilus. NAD is present in whole blood and serum, but its level in these fluids is variable and not stable when in aqueous solution. Therefore, blood and blood products cannot be relied upon to serve as good sources of factor V.

Most blood culture media do not contain adequate levels of these growth factors. This is due to the fact that most blood culture media are sterilized in manufacturing by autoclaving, and since some growth factors, such as factor V, are heat labile, factor V becomes inactivated.

There are a number of available supplements for use in microbiological culture media which are intended to enhance the isolation of fastidious organisms. None of these supplements, however, provides growth factors and neutralizes the toxic effects of polyanionic anticoagulants.

Available supplements for stimulating the growth and recovery of nutritionally fastidious microorganisms include Fildes Enrichment (sold by Becton Dickinson Microbiology Systems, Cockeysville, Maryland), ISOVITALEX® Enrichment (trademark of Becton, Dickinson and Company) sold by Becton Dickinson Microbiology Systems, Cockeysville, Maryland, BACTO® Supplements (trademark of Difco) sold by Difco Laboratories, Detroit, MI and Hemoglobin (sold by Becton Dickinson Microbiology Systems, Cockeysville, Maryland).

Fildes Enrichment is a peptic digest of defibrinated sheep blood containing factor X, and possibly factor V. It is not able to neutralize the toxic effects of polyanionic anticoagulants in culture media.

ISOVITALEX Enrichment, contains factor V, but does not contain factor X and is not able to neutralize the toxic effects of polyanionic anticoagulants in culture media.

The BACTO Supplements include BACTO Supplements A, B, C and VX. BACTO Supplement A is a desiccated yeast concentrate which contains crystal violet, a dye which inhibits growth of many bacteria and is not acceptable as a blood culture medium supplement. BACTO Supplement A preserves the thermolabile and thermostable growth accessory factors, including glutamine, factor V, cocoarboxylase and factor X. BACTO Supplement A is unable to neutralize the toxic effects of polyanionic anticoagulants in culture media.

BACTO Supplement B is a desiccated yeast concentrate which provides growth factors required to support the growth of both Haemophilus and Neisseria species. BACTO Supplement B preserves the thermolabile and thermostable growth accessory factors of fresh yeast, and contains factor V and factor X. BACTO Supplement B is unable to neutralize the toxic effects of polyanionic anticoagulants in culture media.

BACTO Supplement C is a desiccated yeast concentrate which provides growth factors required to support the cultivation of Haemophilus influenzae and Neisseria gonorrhoeae. BACTO Supplement C contains the thermolabile and thermostable growth accessory factors of fresh yeast, including factor V, factor X and cocoarboxylase. BACTO Supplement C is unable to neutralize the toxic effects of polyanionic anticoagulants in culture media.

BACTO Supplement VX is a lyophilized concentrate of growth factors used as an enrichment for the cultivation of Neisseria gonorrhoeae, Haemophilus influenzae and other fastidious organisms. BACTO Supplement VX contains factor V, but it does not contain factor X and is unable to neutralize the toxic effects of polyanionic anticoagulants in culture media.

Hemoglobin provides factor X, is able to neutralize the toxic effects of polyanionic anticoagulants in culture media, but it does not contain factor V.

The following table summarizes the characteristics of available supplements in regard to providing growth factors and polyanionic anticoagulant neutralization, in culture media.

**TABLE 1**

| | **GROWTH FACTORS** | | **POLYANIONIC ANTICOAGULANT NEUTRALIZATION** |
|---|---|---|---|
| | **FACTOR X** | **FACTOR V** | |
| ISOVITALEX | - | + | - |
| FILDES Enrichment | + | var. | - |
| BACTO Supplement XV | - | + | - |
| BACTO Supplement B | + | + | - |
| BACTO Supplement C | + | + | - |
| Hemoglobin | + | - | + |
| var.=variable results | | | |

The available supplements are not able to provide a combination of growth factors and polyanionic anticoagulant neutralization.

Available supplements are for use in plated media or liquid media which normally do not contain polyanionic anticoagulants. Therefore, the available supplements do not contain properties that neutralize the toxic effects of polyanionic anticoagulants.

The available supplements discussed in Table 1, will not allow the growth of fastidious organisms and in particular both Neisseria and Haemophilus species in blood culture medium when cultured from body fluids such as normally sterile body fluids other than blood. Such body fluids (i.e. cerebrospinal fluid, joint fluid, peritoneal fluid) are typically tested using solid, plated media, or nutrient broths other than blood culture media. Plated media cannot accommodate large sample sizes (maximum of about 0.2 ml) and cannot be used with automated blood culturing systems such as the BACTEC® system (trademark of Becton, Dickinson and Company) sold by Becton Dickinson Diagnostic Instrument Systems, Towson, Maryland. Blood culture media, however, may accommodate large sample sizes and can be used with automated blood culturing systems such as the BACTEC system.

Freshly drawn whole blood generally suffices to neutralize the toxic effects of anticoagulants and provide necessary growth factors, but there are many problems and disadvantages in its use. Banked blood (such as outdated blood units, or commercially available sheep blood) sometimes does not contain sufficient amounts of necessary growth factors and therefore cannot be relied upon to support the growth of fastidious organisms, in particular, Haemophilus and Gardnerella strains.

Thus, a special need exists for a fastidious organism supplement which neutralizes the toxic effect of polyanionic anticoagulants and provides necessary growth factors which are often absent or limiting in culture media, and to stimulate the isolation, growth, recovery and/or enhanced detection of fastidious organisms.

### SUMMARY OF THE INVENTION

The present invention is a fastidious organism supplement (FOS) composition comprising:
(a) NAD or a functional substitute thereof;
(b) hemin or a functional substitute thereof;
(c) an albumin or a functional substitute thereof;
(d) a polycationic compound being a water soluble cationic polymer or oligomer; and
(e) an acid and its salt or buffer.

FOS may be used in microbiological cultures, without inhibiting the growth of fastidious organisms.

A preferred supplement of the invention comprises:
(a) NAD;
(b) hemin;
(c) bovine serum albumin;
(d) water-soluble polycationic polymer; and
(e) citric acid.

A further aspect of the invention includes a method for making a fastidious organism supplement as defined above comprises:
(a) freeze drying NAD, hemin and bovine serum albumin in a first vial;
(b) mixing an aqueous reconstituting solution of water-soluble polycationic polymer or oligomer and citric acid in a second vial; and
(c) adding said aqueous reconstituting solution to said freeze dried solution in said first vial.

FOS is useful in stimulating recovery of nutritionally fastidious microorganisms from body fluids such as normally sterile body fluids which have been added to blood culture media. Primary applications include samples of pediatric blood, cerebrospinal fluid and synovial fluid which are tested using blood culture media.

FOS solves the problem of using polyanionic anticoagulants in culture media without inhibiting the growth of organisms which are sensitive to polyanionic anticoagulants.

FOS also solves the problem of providing growth factors which are often absent or limiting in culture media.

An advantage of FOS is that it may be used in culture media methods for only neutralizing the toxic effects of a polyanionic anticoagulant, for only providing growth factors or a combination of both.

A further advantage is that FOS can be used in automated blood culturing systems such as the BACTEC system.

A further advantage of FOS is that it stimulates the growth, recovery, isolation and/or enhanced detection of both Haemophilus and Neisseria species in a culture media which may contain polyanionic anticoagulants.

Another advantage of FOS is that it may be used in many types of culture media such as plated media and preferably in blood culture media.

### DETAILED DESCRIPTION

Nicotinamide adenine dinucleotide (NAD) is a complex organic molecule and is useful for the growth of a number of fastidious organisms. The functional substitute for NAD includes, but is not limited to, β-nicotinamide adenine dinucleotide (β-NADH) (reduced form of NAD), β-nicotinamide adenine dinucleotide phosphate (reduced β-NADP or oxidized β-NADPH form of NADP), and in general any form of the related chemical which serves as a functional substitute for NAD, including all salt forms of these compounds. A desirable compound which serves as a functional substitute for NAD is β-NADH and the preferred compound is NAD.

Hemin is a compound present in red blood cells and is useful for the growth of a number of fastidious organisms. The functional substitute for hemin includes, but is not limited to, hemoglobin, myoglobin, protoporphyrin, hematin, heme, protoheme and in general any related compounds which serve as a functional substitute for hemin. A desirable compound which serves as a functional substitute for hemin is hemoglobin and the preferred compound is hemin.

An albumin has the capability to interact with and neutralize the toxicity of a polyanionic anticoagulant. The functional substitute for albumin includes, but is not limited to, bovine serum albumin, serum albumin, gelatin, globulins, soluble proteins, digests of proteins, free amino acids, arginine, histidine, lysine, choline salt, guanidine salt, water soluble cationic chemicals and protamine sulfate. A desirable albumin is serum albumin and the preferred is bovine serum albumin.

Polycationic compounds also interact with and neutralize the toxicity of polyanionic anticoagulants. Since polycationic compounds are positively charged, polyanionic anticoagulants which are anionic molecules (negatively charged), will bind to polycationic compounds.

The polycationic compound comprises water soluble polycationic polymers or oligomers. A desirable polycationic compound is GAFQUAT® 755N polymer (trademark of GAF, Wayne, NJ) sold by Serva Biochemicals, Inc. of Montvale, New Jersey. GAFQUAT 755N polymer is a vinylpyrrolidone (1-ethenyl-2-pyrrolidinone; CAS registry no.: 00053633-54-8).

An acid and its salt or buffer allow the pH of certain components in a composition to be adjusted to produce optimum clarity and stability. An acid and its salt or buffer include, but is not limited to nonvolatile pH adjusting chemicals. Such chemicals include, but are not limited to, dicarboxylic acids and tricarboxylic acids. Dicarboxylic acids include, but are not limited to, malic acid, tartaric acid, mandelic acid, succinic acid, and fumaric acid. Tricarboxylic acid includes, but is not limited to, citric acid. A desirable acid is a tricarboxylic acid and the preferred acid is citric acid.

The desired pH of the FOS composition is from 1.5 to 4.0, while the preferred pH is from to 2.0 3.5 and the most preferred pH is 2.5.

FOS preferably may be used to stimulate the isolation of fastidious organisms such as, but not limited to, Neisseria meningitidis, Neisseria gonorrhoeae, Peptostreptococcus anaerobius, Streptobacillus moniliformis, Gardnerella vaginalis, Mycoplasma sp., Haemophilus influenzae, Haemophilus parainfluenzae and other strains of Haemophilus, Neisseria and Gardnerella.

The isolation of the fastidious organisms includes their growth, recovery and/or enhanced detection in culture media.

In an even more preferred embodiment of the invention, FOS comprises:
(a) NAD;
(b) hemin;
(c) bovine serum albumin;
(d) a vinylpyrrolidone polymer, such as GAFQUAT 755N; and
(e) citric acid.

FOS may be packaged in several configurations, wherein, all components are preferably sterile in their packaging and use. An illustration of four alternative packaging configurations are as follows:
1. All components of FOS are freeze dried and sterile water is used to reconstitute the components.
2. NAD or a compound which is the functional substitute for NAD is freeze dried along with any combination of the other components from none to all. Any component not freeze dried is in the aqueous solution and serves as the reconstituting fluid.
3. All components of FOS in solution are stored frozen or refrigerated.
4. All components of FOS are in aqueous solution.

The above configurations are examples and are not the only possible ways in which FOS can be packaged. A preferable determinant of the FOS packaging configuration is to have NAD or a compound which is the functional substitute for NAD dried, frozen or refrigerated. FOS may be stored at refrigerator temperatures, from 2 to 6° C, wherein under those conditions, the FOS is stable for several weeks.

FOS preferably may be used in a method which stimulates the isolation of fastidious organisms, in a culture media which lacks certain growth factors and/or which contains a polyanionic anticoagulant.

Polyanionic anticoagulants include, but are not limited to, SPS and SAS.

FOS is preferably used in blood culture media when used to culture body fluids, such as normally sterile body fluids, other than blood. Such body fluids (i.e. cerebral spinal fluid, joint fluid, peritoneal fluid) are typically tested using solid, plated media, or nutrient broths other than blood culture media.

FOS is preferably used in automated blood culturing systems such as the BACTEC system.

FOS is desirably used in a method for providing growth factors in culture media.

FOS is preferably used in a method for neutralizing the toxic effects of a polyanionic anticoagulant in culture media.

FOS is most preferably used in a method for neutralizing a polyanionic anticoagulant and for providing growth factors in culture media.

## Claims

1. A fastidious organism supplement comprising:
(a) NAD or a functional substitute thereof;
(b) hemin or a functional substitute thereof;
(c) an albumin or a functional substitute thereof;
(d) a polycationic compound being a water soluble cationic polymer or oligomer; and
(e) an acid and its salt or buffer.

2. The supplement of claim 1 wherein the functional substitute of NAD is selected from β-NAD, β-NADP and β-NADPH.

3. The supplement of claim 1 wherein the functional substitute of hemin is selected from hemoglobin, myoglobin, protoporphyrin, hematin, heme and protoheme.

4. The supplement of claim 1 wherein albumin or a functional substitute thereof is selected from serum albumin, bovine serum albumin, gelatin, globulins, soluble proteins, digests of proteins, free amino acids, arginine, histidine, lysine, water soluble cationic chemicals, choline, quanidine salts and protamine sulfate.

5. The supplement of claim 1 wherein said acid is a dicarboxylic or tricarboxylic acid.

6. The supplement of claim 1 having a pH of from 1.5 to 4.0.

7. The supplement of claim 1 comprising:
(a) NAD;
(b) hemin;
(c) bovine serum albumin;
(d) water-soluble polycationic polymer; and
(e) citric acid.

8. The supplement of claim 7 wherein the components have the following concentrations in percent weight per volume:
(a) 0.10 NAD;
(b) 0.0015 hemin;
(c) 7.5 bovine serum albumin;
(d) 1.65 water-soluble polycationic polymer; and
(e) 0.42 citric acid.

9. A method for making a fastidious organism supplement according to any of claims 1-8 comprising:
(a) freeze drying NAD, hemin and bovine serum albumin in a first vial;
(b) mixing an aqueous reconstituting solution of water-soluble polycationic polymer or oligomer and citric acid in a second vial;
and (c) adding said aqueous reconstituting solution to said freeze dried solution in said first vial.

## Patentansprüche

1. Ergänzung für anspruchsvolle Organismen, umfassend:
(a) NAD oder einen funktionellen Ersatz dafür;
(b) Hämin oder einen funktionellen Ersatz dafür;
(c) ein Albumin oder einen funktionellen Ersatz dafür;
(d) eine polykationische Verbindung, bei der es sich um ein wasserlösliches kationisches Polymer oder Oligomer handelt; sowie
(e) eine Säure und ihr Salz oder einen Puffer.

2. Ergänzung gemäß Anspruch 1, wobei der funktionelle Ersatz für NAD aus β-NAD, β-NADP und β-NADPH ausgewählt ist.

3. Ergänzung gemäß Anspruch 1, wobei der funktionelle Ersatz für Hämin aus Hämoglobin, Myoglobin, Protoporphyrin, Hämatin, Häm und Protohäm ausgewählt ist.

4. Ergänzung gemäß Anspruch 1, wobei das Albumin oder der funktionelle Ersatz dafür aus Serumalbumin, Rinderserumalbumin, Gelatine, Globulinen, löslichen Proteinen, Spaltprodukten von Proteinen, freien Aminosäuren, Arginin, Histidin, Lysin, wasserlöslichen kationischen Chemikalien, Cholin, Guanidinsalzen und Protaminsulfat ausgewählt ist.

5. Ergänzung gemäß Anspruch 1, wobei die Säure eine Dicarbonsäure oder eine Tricarbonsäure ist.

6. Ergänzung gemäß Anspruch 1, die einen pH-Wert von 1,5 bis 4,0 aufweist.

7. Ergänzung gemäß Anspruch 1, umfassend:
(a) NAD;
(b) Hämin;
(c) Rinderserumalbumin;
(d) ein wasserlösliches polykationisches Polymer; sowie
(e) Zitronensäure.

8. Ergänzung gemäß Anspruch 7, wobei die Komponenten die folgenden Konzentrationen in Gew.-% pro Volumen haben:
(a) 0,10 NAD;
(b) 0,0015 Hämin;
(c) 7,5 Rinderserumalbumin;
(d) 1,65 des wasserlöslichen polykationischen Polymers; sowie
(e) 0,42 Zitronensäure.

9. Verfahren zur Herstellung einer Ergänzung für anspruchsvolle Organismen gemäß einem der Ansprüche 1 bis 8, umfassend:
(a) Gefriertrocknen von NAD, Hämin und Rinderserumalbumin in einem ersten Gläschen;
(b) Mischen einer wäßrigen rekonstituierenden Lösung eines wasserlöslichen polykationischen Polymers oder Oligomers und Zitronensäure in einem zweiten Gläschen; und
(c) Hinzufügen der wäßrigen rekonstituierenden Lösung zu der gefriergetrockneten Lösung in dem ersten Gläschen.

## Revendications

1. Supplément pour germes exigeants comprenant:
(a) du NAD ou un de ses équivalents fonctionnels;
(b) de l'hémine ou un de ses équivalents fonctionnels;
(c) une albumine ou un de ses équivalents fonctionnels;
(d) un composé polycationique qui est un polymère ou un oligomère cationique hydrosoluble; et
(e) un acide et son sel ou tampon.

2. Supplément selon la revendication 1, dans lequel l'équivalent fonctionnel du NAD est choisi parmi le β-NAD, le β-NADP et le β-NADPH.

3. Supplément selon la revendication 1, dans lequel l'équivalent fonctionnel de l'hémine est choisi parmi l'hémoglobine, la myoglobine, la protoporphyrine, l'hématine, l'hème et le protohème.

4. Supplément selon la revendication 1, dans lequel l'albumine ou l'un de ses équivalents fonctionnels est choisi parmi la sérum albumine, la sérum albumine bovine, la gélatine, les globulines, des protéines solubles, des produits de digestion de protéines, des acides aminés libres, l'arginine, l'histidine, la lysine, des substances chimiques cationiques hydrosolubles, la choline, les sels de guanidine et le sulfate de protamine.

5. Supplément selon la revendication 1, dans lequel ledit acide est un acide dicarboxylique ou tricarboxylique.

6. Supplément selon la revendication 1 ayant un pH de 1,5 à 4,0.

7. Supplément selon la revendication 1 comprenant:
(a) du NAD;
(b) de l'hémine;
(c) de la sérum albumine bovine;
(d) un polymère polycationique hydrosoluble; et
(e) de l'acide citrique.

8. Supplément selon la revendication 7, dans lequel les composants ont les concentrations suivantes en pourcentage pondéral par volume:
(a) NAD 0,10;
(b) hémine 0,0015;
(c) sérum albumine bovine 7,5;
(d) polymère polycationique hydrosoluble 1,65; et
(e) acide citrique 0,42.

9. Procédé de préparation d'un supplément pour germes exigeants selon l'une quelconque des revendications 1-8, comprenant les étapes qui consistent à:
(a) lyophiliser le NAD, l'hémine et la sérum albumine bovine dans un premier flacon;
(b) mélanger une solution aqueuse de reconstitution de polymère ou d'oligomère polycationique hydrosoluble et d'acide citrique dans un deuxième flacon; et
(c) ajouter ladite solution aqueuse de reconstitution à ladite solution lyophilisée dans ledit premier flacon.
